# EUROPEAN PATENT SPECIFICATION

(11) **EP 2 297 662 B1**
(45) Date of publication and mention of the grant of the patent: **07.08.2019**
(21) Application number: 09738570.2
(22) Date of filing: 28.04.2009
(51) Int. Cl.: G16H 20/17

(54) **METHODS AND APPARATUSES FOR SELECTING A BOLUS DELIVERY PATTERN IN A DRUG DELIVERY DEVICE**
VERFAHREN UND VORRICHTUNGEN ZUR BOLUS-DOSIS UND -VERABREICHUNGSAUSWAHL IN EINER ARZNEIMITTELABGABEVORRICHTUNG
PROCÉDÉS ET APPAREILS POUR SÉLECTIONNER UN MOTIF D'ADMINISTRATION DE BOLUS DANS UN DISPOSITIF D'ADMINISTRATION DE MÉDICAMENT

(30) Priority: 29.04.2008 US 48849
(43) Date of publication of application: 23.03.2011
(73) Proprietor: Roche Diabetes Care GmbH, 68305 Mannheim (DE); F. Hoffmann-La Roche AG, 4070 Basel (CH)
(72) Inventor: YODFAT, Ofer, 71908 Maccabim-Reut (IL); GESCHEIT, Iddo M., 69395 Tel-Aviv (IL); SHAPIRA, Gali, 34558 Haifa (IL)
(74) Representative: Evens, Paul Jonathan
(86) International application number: PCT/IL2009/000453
(87) International publication number: WO 2009/133557

(56) References cited:
- WO-A2-00/10628
- WO-A2-2007/000425
- US-A1- 2003 163 088
- US-A1- 2005 065 760
- US-A1- 2007 112 298

## Description

### RELATED APPLICATION

The present application claims priority to U.S. provisional application no. 61/048,849, filed April 29, 2008.

### FIELD

Methods, systems and devices for sustained medical infusion of fluids are described. Some implementations describe portable infusion methods, systems and devices for selecting a pattern of fluid delivery. Some implementations describe skin securable insulin dispensing methods, systems and devices for selecting a delivery pattern according to the nutritional characteristics (e.g. glycemic index, GI, fat content, etc.) of caloric intake of the user.

### BACKGROUND

Diabetes mellitus is a disease of major global importance, increasing in frequency at almost epidemic rates, such that the worldwide prevalence in 2006 is 170 million people and predicted to at least double over the next 10-15 years. Diabetes is characterized by a chronically raised blood glucose concentration (hyperglycemia), due to a relative or absolute lack of the pancreatic hormone, insulin. Within the healthy pancreas, beta cells, located in the islets of Langerhans, continuously produce and secrete insulin according to the blood glucose levels, maintaining near constant glucose levels in the body.

Much of the burden of the disease to the patient and to health care resources is due to the long-term tissue complications, which affect both small blood vessels (microangiopathy, causing eye, kidney and nerve damage) and large blood vessels (causing accelerated atherosclerosis, with increased rates of coronary heart disease, peripheral vascular disease and stroke). The Diabetes Control and Complications Trial (DCCT) demonstrated that development and progression of the chronic complications of diabetes are greatly related to the degree of altered glycemia as quantified by determinations of glycohemoglobin (HbA1c). [DCCT Trial, N Engl J Med 1993; 329: 977-986, UKPDS Trial, Lancet 1998; 352: 837-853. BMJ 1998; 317, (7160): 703-13 and the EDIC Trial, N Engl J Med 2005; 353, (25): 2643-53]. Thus, maintaining euglycemia by frequent glucose measurements and adjustment of insulin delivery accordingly is of utmost importance.

Insulin pumps have been available which deliver rapid acting insulin 24 hours a day through a catheter placed under the skin. The total daily insulin dose can be divided into basal and bolus doses. Basal insulin is delivered continuously over 24 hours, and keeps blood glucose levels in an acceptable range between meals and overnight. Diurnal basal rates can be pre-programmed or manually changed according to various daily activities.

Insulin boluses are delivered before or after meals to counteract carbohydrates loads or during episodes of high blood sugar levels. The amount of insulin in the administered insulin bolus depends on several parameters:
- the amount of carbohydrates (Carbohydrates) in the meal to be consumed;
- carbohydrate-to-insulin ratio (CIR), i.e. the amount of carbohydrates balanced by one unit of insulin;
- insulin sensitivity (IS), i.e., the amount of blood glucose lowered by one unit of insulin;
- current blood glucose level (CBG);
- target blood glucose level (TBG), i.e., the desired blood glucose level -- TBG for most people suffering from diabetes is in the range of 90-130 mg/dL; and
- remaining insulin, i.e., the amount of stored insulin remained active in the body after recent boluses have been delivered. This parameter is relevant when there is a short time interval between delivering consecutive boluses (e.g. time interval less than 5 hours).

The amount of insulin in the bolus to be delivered can be defined either by calculations using equations that include the above-mentioned parameters, as described in U.S. Patent 6,936,029 assigned to Medtronic MiniMed, or they can be selected by a method for selection of the desired bolus dose, as described in co-owned, co-pending U.S. Patent Application No. 12/051,400 published as US2008/0234663, and International Patent No. PCT/IL2008/000380, published as WO2008/114254.

The bolus delivery pattern, which will be referred-to further simply as "bolus pattern" or "pattern" refers to a rate or rates at which the bolus dose is administered to the patient over time. For example, a pure carbohydrate containing meal requires a very fast delivery at a constant rate (single delivery rate). A meal containing carbohydrates and fat (e.g., a "Pizza meal") requires a fast delivery rate for a short time followed by a slower delivery rate for an extended period (two delivery rates).

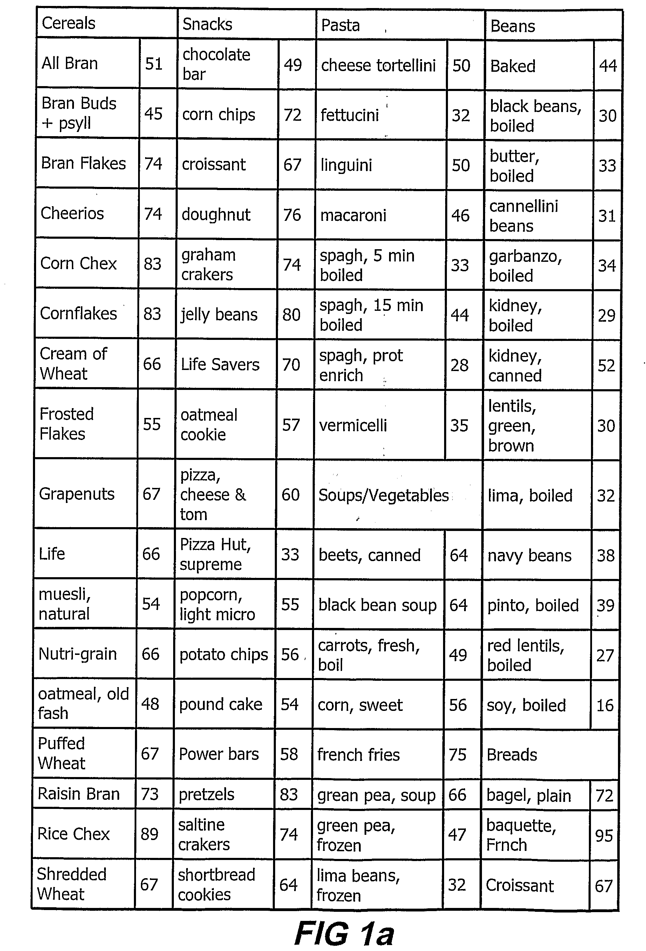

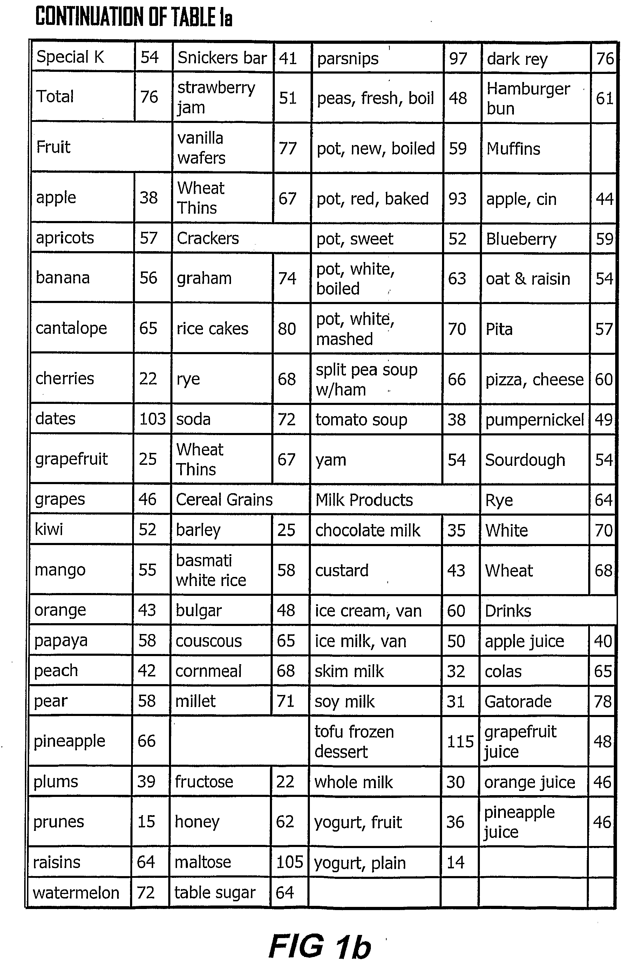

Certain parameters, such as glycemic index (GI) or fat content of the caloric intake of the user (hereinafter either "caloric intake", "food intake", or "intake"), can influence carbohydrates absorption and, consequently, bolus delivery time and pattern. The GI can be represented by a ranking system attributing the carbohydrates contained in food according to their ability to affect the blood glucose levels. According to the GI, glucose (the fastest-acting carbohydrate) is given a value of 100, and the other carbohydrates are ranked relative to that value. Ripeness, cooking time, fiber, and fat content in the food can all impact the glycemic index of certain foods. For example, FIG. 1 (acquired from http://www.diabetesnet.com/diabetes_tools/glycemic_index.php) depicts different types of foods and their GI.

A low GI food will release glucose more slowly and steadily. A high GI food causes a more rapid rise in blood glucose. Accordingly, a meal containing a carbohydrate load having a high GI would require immediate delivery of insulin to counteract the carbohydrates rapidly absorbed through the gut. A meal containing a carbohydrate load having a low GI would require insulin to be delivered over a long period of time to counteract the slowly absorbed carbohydrates.

Currently, most insulin delivery pumps allow the user to program a bolus delivery pattern before insulin administration. The most common programmable delivery patterns are:
1. immediate ("regular", "normal") bolus - the entire bolus dose is delivered at the fastest pump delivery rate;
2. extended bolus - the entire bolus is delivered over a long period of time (e.g. 30 min-8 hours) at a constant rate; and
3. combined bolus ("dual wave") - some of the bolus dose is delivered as an immediate bolus and the rest of the bolus dose is delivered as an extended bolus. Usually the ratio (in percentage) between the immediately delivered bolus portion and the extended bolus portion can be selected by the patient, e.g. as described in US patent No. US 6,852,104 assigned to Smiths Medical (formerly Deltec Medical).

In the current devices, the user programs the desired bolus delivery pattern by inputting one or more of the following data: duration of bolus delivery (for extended bolus and for extended portion of combined bolus), ratio between the immediate bolus portion and extended bolus portion (for combined bolus).

A drawback associated with the user's preprogramming of currently existing bolus patterns is the user's inability to properly estimate bolus delivery duration and adequate bolus delivery pattern. In practice, user's decision about the bolus pattern often is arbitrary and based merely on intuition.

Additional drawbacks associated with preprogramming of bolus delivery patterns include:
- the necessity for data input which complicates the user interface because it requires navigation through several displays which are not user-friendly;
- young children having difficulties in mastering the data input since it is associated with reading and typing alpha-numeric parameters; and
- available bolus programming features are not provided with graphic representation of the available bolus patterns and instead require inputting quantitative alpha numeric parameters. In result, the programming process might cause frustration and it usually paradoxically results in administration of insulin according to merely normal bolus delivery pattern irrespective of the meal type. Such regrettably rather common situation might be associated with even worse glycemic control, with elevated HbAlc values and eventually with diabetes complications.

WO 2007/000425 relates to a medical delivery device with a user interface for controlling the medical delivery device, and discloses a drug delivery device according the preamble of appended independent claim 1, together with a bolus delivery pattern selection method according to the preamble of appended independent claim 12.

US 2005/0065760 relates to a method of advising patients concerning doses of insulin.

US 2003/0163088 relates to a programmable medical infusion pump.

US 2007/0112298 relates to an external infusion device with programmable capabilities to time-shift basal insulin, and a method of using the same.

WO 00/10628 relates to an external infusion device with remote programming, bolus estimator and/or vibration alarm capabilities.

### SUMMARY

In accordance with one aspect of the present invention, there is provided a drug delivery device as defined in appended independent claim 1. Embodiments of the invention are defined in appended claims which depend on independent claim 1. In accordance with another aspect of the present invention, there is provided a bolus delivery pattern selection method as defined in appended independent claim 12. Methods and devices are provided for selecting a preprogrammed bolus delivery pattern of a drug. According to some embodiments, there is provided a drug delivery device comprising : a first module adapted for receiving a drug bolus to be delivered; a memory, the memory being adapted for storing a plurality of pre-programmed bolus delivery patterns, with each bolus delivery pattern defining the rate or rates at which the drug bolus is administered to the user; a dispensing unit adapted for delivering the received drug bolus to the user; a screen adapted for providing to a user a graphical representation of each of the plurality pre-programmed bolus delivery patterns in accordance with the received drug bolus to be delivered; and a second module adapted for receiving a user selection identifying the graphical representation of one of the plurality of pre-programmed bolus delivery patterns; the dispensing unit being configured to deliver the received drug bolus to the user at a drug delivery rate in accordance with the pre-programmed bolus delivery pattern of the graphical representation selected by the user; characterized in that: the plurality of pre-programmed bolus delivery patterns are adapted to meals containing different foods having more than one glycemic index; and at least one of the plurality of pre-programmed bolus delivery patterns is recommended to the user for selection based upon inputted parameters associated with the user which are in accordance with nutritional characteristics of a contemplated meal comprising different foods having more than one glycemic index, with the glycemic index of the different foods of the contemplated meal being entered one after the other.

In some embodiments, there is provided a bolus delivery pattern selection method for selecting a bolus delivery pattern for delivering a drug bolus to a user by a drug delivery device, the method comprising: providing a drug delivery system, including a drug infusion device and optionally including a remote control device to send and receive information and/or instructions to the drug infusion device, wherein at least one of the drug delivery device and the remote control device includes a display; storing a plurality of pre-programmed bolus delivery patterns in a memory of at least one of the remote control unit and the drug infusion device, with each bolus delivery pattern defining the rate or rates at which the drug bolus is administered to the user; providing to a user, via the display, a graphical representation of each of the plurality of pre-programmed bolus delivery patterns, in accordance with the received amount of drug bolus; receiving a user selection via at least one of the remote control device and the drug infusion device, identifying one of the plurality of pre-programmed bolus delivery patterns; and determining a drug delivery rate for delivering the drug bolus to the user in accordance with the bolus delivery pattern identified by the user selection; characterized in that: the plurality of programmed pre-programmed bolus delivery patterns are adapted to meals containing different foods having more than one glycemic index; and at least one of the plurality pre-programmed bolus delivery patterns is recommended to the user for selection based upon inputted parameters associated with the user which are in accordance with nutritional characteristics of a contemplated meal comprising different foods having more than one glycemic index, with the glycemic index of the different foods of the contemplated meal being entered one after the other.

In some embodiments, the drug is insulin and the dispensing unit is an insulin pump. The graphical representation of the recommended bolus delivery pattern can be visually distinct from at least one other graphical representation of a bolus delivery pattern. The at least one graphical representation of a bolus delivery pattern corresponds to at least two different delivery rates.

In addition, according to some embodiments, the displayed graphical representation of a bolus delivery pattern (as noted above) can correspond to the correction of the blood glucose value before a contemplated meal.

In some embodiments, the drug delivery device can further comprise a user interface adapted for modification of the bolus delivery duration corresponding to the at least one pre-configured bolus delivery pattern. Some embodiments may also include a glucometer, and a remote control unit, where the glucometer can be provided in the remote control unit.

In some embodiment, a drug delivery device can also comprise a dispensing patch unit, and a glucometer can be provided therein. Moreover, in some embodiments, the drug delivery device may also comprise a continuous glucose monitor (CGM), which can be provided in the dispensing patch unit. Such a device is disclosed for example in co-owned, co pending U.S. Patent Application Nos. 11/706,606, 11/963,481 and International Patent Application No. PCT/IL2008/001521, published as WO 2009/066288.

In some embodiments, the drug delivery device can be adapted for at least one of: storing at least one selected bolus delivery pattern, and storing a point in time corresponding to a delivery of the bolus in accordance with the selected bolus delivery pattern. In one implementation, a graphical representation of the at least one pre-programmed bolus delivery pattern can correspond to the at least one stored selected bolus delivery patterns.

In some embodiments which are provided with graphical representations of pre-programmed bolus delivery patterns, recommendations for selection by the user can be in accordance with at least one stored point in time corresponding to a delivery of the bolus in accordance with the selected bolus delivery pattern.

In some arrangements, operation of a fluid delivery device can be carried out manually by operating buttons/switches located on the dispensing patch unit. For example, in one arrangement, the delivery device may be a dispensing patch unit which can be composed of two parts - a disposable part and a reusable part. The disposable part can contain a reservoir, outlet port and other inexpensive components. The reusable part can contain electronics (PCB, processor, etc), driving mechanism and other relatively expensive components.

In some arrangements, a fluid delivery system is provided which may include a cradle unit to receive a fluid delivery device (according to any of the noted fluid delivery device arrangements, for example). The cradle unit can be a substantially flat sheet or element that adheres to the skin and allows disconnection and reconnection of the fluid delivery device (e.g., a patch unit) from and to the patient skin upon patient discretion. After attachment of the cradle unit to the skin, a cannula for insulin delivery can be inserted into a subcutaneous compartment of the patient through a dedicated passageway in the cradle unit.

In some arrangements, the user can select a preprogrammed delivery pattern out of several (e.g., more than one) delivery patterns that are presented on a screen provided on the remote control unit or on the drug delivery device (e.g., patch unit). For example, selectable bolus delivery patterns can be the most common four patterns as explained below (for example):
1. the entire bolus dose to be delivered immediately at a single, constant delivery rate;
2. a portion of the bolus dose to be delivered immediately at a first delivery rate and the rest over 2 hours at a second delivery rate;
3. a portion of the bolus dose to be delivered immediately at a first delivery rate and the rest over 4 hours at a second delivery rate; and
4. an entire dose to be delivered over 6 hours at a single, constant delivery rate.

For some arrangements, restricting the available preprogrammed bolus delivery patterns merely to those associated with the most common cases makes the selection easier and at the same time still convenient and adequate.

In some arrangements, a fluid delivery device is provided that can also monitor glucose concentration levels (e.g., in blood) and can also dispense insulin according to a bolus delivery pattern which can be selected via a simplified, easy to use bolus delivery pattern selecting method (for example). In some such arrangements, a fluid delivery device may be provided that continuously monitors body glucose levels and can concomitantly deliver insulin according to a bolus delivery pattern selected by virtue of a simplified, easy to use bolus delivery pattern selecting method (for example).

In some arrangements, a fluid delivery system includes a fluid delivery device that may comprise a skin securable dispensing patch unit which may include two parts. The system may further include a cradle unit which is adhered to the skin of the patient and which receives the fluid delivery device (can be connected and disconnected therefrom). The patch unit (and/or system) may also include a simplified, easy to use method to select an insulin bolus delivery pattern.

In some arrangements, a fluid delivery system includes a fluid delivery device comprising a dispensing patch unit that can be disconnected and reconnected to a patient, where the device employs a simplified, easy to use method to select an insulin bolus delivery pattern. Such arrangements may include a miniature skin securable patch unit that can continuously dispense insulin, monitor body glucose concentration levels, and include a simplified, easy to use method to select an insulin bolus delivery pattern.

Some arrangements of the present disclosure may provide a semi closed loop insulin dispensing system, which may be a miniature/micro-sized device that can monitor glucose levels and dispense insulin according to at least one of sensed glucose levels and a selected bolus delivery pattern. The bolus delivery pattern may be selected by a simple method.

Some arrangements include a fluid delivery system/device that is configured as an insulin infusion patch unit comprising a disposable part and a reusable part. The reusable part contains all relatively expensive components and the disposable part contains inexpensive components, thus providing a low cost product for the user and a highly profitable product for the manufacturer and payer. The device may implement a simplified, easy to use method for selecting a bolus delivery pattern.

Some arrangements may provide a fluid delivery device that comprises an insulin infusion patch unit that can be remotely controlled.

The method(s) for selecting a suitable insulin bolus delivery pattern is based on data that takes into account at least the GI of the caloric intake of the user. The suitable insulin bolus delivery pattern can be selected from a plurality of preprogrammed bolus delivery patterns, each pattern being assigned to a different range of GIs (e.g. GI<55, 56<GI<69, GI>70). The GI or indexes of the intake may be defined also by a qualitative, descriptive parameter (QDP) (e.g. high, intermediate, low GI, or combined GI). Presenting GI of the intake as a qualitative parameter instead of a number would be especially convenient for young children.

In some arrangements, one or more previously selected bolus delivery patterns can be stored in a memory of a bolus delivery pattern selection feature. For example, the most prevalent delivery pattern selected during a specific time interval can be established and presented. Thus, selection of a bolus delivery pattern can be carried out according to the stored prevalent bolus delivery pattern and the selection can be done, for example, even prior to loading of a GI of the intake. The most prevalent delivery pattern can be presented as a first preferable choice, for example. This feature may be especially beneficial for the users with routine daily intakes. In some arrangements, the prevalent delivery pattern can be presented to the user only if the prevalence is statistically significant (e.g. over 70% of the time).

In some arrangements, a user can accept a recommended bolus delivery pattern, select it and allow delivery of the corresponding bolus accordingly. The selected bolus delivery pattern may be delivered upon confirmation by the user through a user interface. In some instances, the user may be notified prior to bolus administration, either to confirm or suspend the delivery, or select an alternative bolus delivery pattern.

The bolus delivery pattern selection method according to some arrangements can be implemented in an insulin infusion system comprising an insulin infusion device (for example), which may comprise an insulin dispensing patch unit, and a remote control unit. Such a system may also include a glucose sensing apparatus (e.g. glucometer), which may be integrated in the remote control unit (and/or the patch unit). The bolus delivery pattern selection feature can be implemented in the remote control unit (for example), and/or in a reusable part of the dispensing patch unit of the infusion device.

In some arrangements, the bolus delivery pattern selection feature can be implemented in the dispensing patch unit that continuously monitors body glucose levels and can, for example, concomitantly deliver insulin into the body. As previously noted, in some arrangements, the dispensing patch unit may comprise a reusable part and a disposable part. The insulin dispensing and glucose sensing capabilities can also be combined into a semi-closed loop system, where a processor-controller regulates the dispensing of basal insulin according to the sensed glucose concentration. The meal boluses may be delivered in accordance with the bolus delivery profile selected by the user.

In some arrangements, the recommended bolus delivery pattern can be selected from a plurality of preprogrammed bolus delivery patterns according to the nutritional composition (e.g., amount or percentage of fat, protein and/or carbohydrate) of the intake.

In some arrangements, the insulin infusion device can deliver a bolus dose in accordance with a delivery pattern selected from a plurality of preprogrammed bolus delivery patterns and can also simultaneously receive glucose levels readings. In some such arrangements, the dispensing device can continuously monitor glucose levels and can deliver a bolus dose in accordance with a delivery pattern selected from a plurality of preprogrammed bolus delivery patterns.

In some arrangements, a dispensing patch unit which implements a bolus delivery pattern selection method can be disconnected from and reconnected to the patient. For example, in some such arrangements, disconnections and reconnections preferably do not harm various components of the patch, like (for example) the pumping mechanism, the needle, nor the surrounding tissue of the patient.

In some arrangements, a drug delivery system can include a dispensing patch unit and a cradle unit. The cradle unit can be adherable to the skin, and the dispensing patch unit can be connected to and disconnected from the cradle unit upon patient discretion.

In some arrangements, the bolus delivery pattern selection method can be implemented in a device that comprises a remotely controllable insulin infusion patch unit. For example, the bolus delivery pattern selection method can be implemented in a remote control unit for an insulin dispensing device in an insulin dispensing system. The bolus delivery pattern selection method can be implemented in the insulin dispensing device that can also comprise a bolus dose selection feature. The bolus delivery pattern selection method can also be implemented in a device comprising a miniature skin securable patch that can continuously dispense insulin and monitor body glucose concentration levels.

In some arrangements the drug delivery system can be configured as a semi-closed loop system, e.g., a system which includes a device that monitors glucose levels and dispenses insulin according to the sensed glucose levels and according to the selected the bolus delivery pattern.

The above noted exemplary embodiments and features of the present disclosure will be even better understood by reference to the attached drawings, a brief description of which is provided below, and subsequent detailed description section.

### BRIEF DESCRIPTION OF THE DRAWINGS

FIG. 1 is a chart of different types of foods and their corresponding GI.
FIG. 2 illustrates an insulin infusion system comprising an insulin dispensing unit and a remote control unit that can be provided with a bolus delivery pattern selection feature, according to some arrangements of the present disclosure.
FIGs. 3a-e illustrate examples of different bolus delivery patterns that can be suggested to the user by the bolus delivery pattern selection feature according to some arrangements of the present disclosure.
FIG 4 illustrates a remote control unit that can be provided with a bolus delivery pattern selection feature according to some arrangements of the present disclosure.
FIGs. 5a-d illustrate examples of a user interface for using the bolus delivery pattern selection feature according to some arrangements of the present disclosure.
FIGs 6 a-c illustrate different display windows displaying exemplary explanation of some meal contents and their relevance to each bolus delivery pattern, according to some arrangements of the present disclosure.
FIGs. 7a-h illustrate an example of a display flow of displays (hereinafter may also be referred to as "graphical representations") for an example of a bolus delivery pattern selection feature according to some embodiments of the present disclosure.
FIGs. 8a-h illustrate another example of a display flow of displays for an example of a bolus delivery pattern selection feature according to some embodiments of the present disclosure.
FIGs. 9a-h illustrate another example of a display flow of displays of a bolus delivery pattern selection feature according to some embodiments of the present disclosure.
FIGs. 10a-c illustrate exemplary arrangements of an insulin infusion system, which may comprise an insulin dispensing unit and a remote control unit, where either or both of the dispensing unit and remote control unit can be provided with a bolus delivery pattern selection feature, and/or a glucometer, for example, according to some arrangements of the present disclosure.
FIGs. 11a-b illustrate exemplary arrangements of an insulin infusion system which may comrpise an insulin dispensing unit and a remote control unit, where either or both of the dispensing unit and remote control unitcan be provided with a bolus delivery pattern selection feature, and/or a continuous subcutaneous glucose monitor, for example, according to some arrangements of the present disclosure.

### DETAILED DESCRIPTION

Methods, systems and devices are provided for selecting a bolus delivery pattern of a drug in drug/medical fluid dispensing. In some arrangements, a method for selecting a bolus delivery pattern of a drug for a drug delivery system/device is described. For example, in one such arrangement, a drug delivery device may comprise a memory adapted for storing at least one pre-programmed bolus delivery pattern (for example), a first module adapted for receiving a drug bolus to be delivered (for example), a screen adapted for providing a graphical representation of the at least one pre-programmed bolus delivery pattern to a user (for example), a second module adapted for receiving a user selection identifying the graphical representation of the at least one bolus delivery pattern (for example), and a dispensing unit adapted for delivering of the drug bolus to the user at a drug delivery rate in accordance with the selected bolus delivery pattern.

It should be noted, that for the purposes of this application, the phrase "drug delivery device", "fluid delivery device", "insulin infusion pump" can refer to a device capable of supplying a therapeutic fluid to a mammal's body at a controllable rate. In some implementations, this device can be a single physical unit, and/or may comprise multiple components (e.g. a patch unit comprising a two-piece design). The phrase "drug delivery system", "insulin infusion system", and "fluid delivery system" can refer to a system which comprises a "drug delivery device", a "fluid delivery device", and/or "insulin infusion pump", as well other devices, including, for example, a remote control unit.

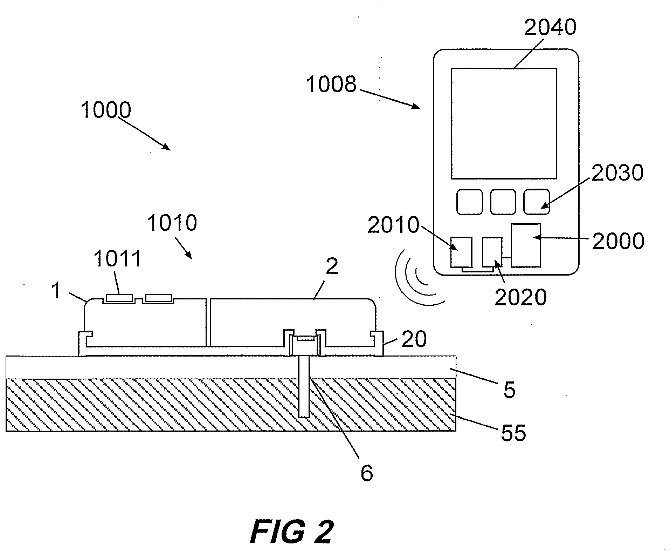

FIG. 2 illustrates an example of a fluid delivery system, e.g. an insulin infusion system (1000) comprising a dispensing patch unit (1010), which can be secured to the user's skin (5), above the subcutaneous tissue (55), and a remote control unit (1008), which can communicate with the dispensing patch unit (1010), allowing programming, user inputs and data acquisition.

The patch unit (1010) can be detachable from the skin. It can also receive a cannula (6) that penetrates the skin (5) to allow delivery of insulin to the patient. The patch unit (1010) can be attached either immediate to the skin or to a dedicated cradle unit (20) that can be a flat sheet adherable to the user's skin (5) and can allow connection/disconnection of the patch unit (1010). An exemplary arrangement is discussed in a co-owned, co-pending U.S. Patent Application No. 12/004,837 and International Patent Application No. PCT/IL2007/001578, published as WO 2008/078318.

Manual inputs for operating and controlling the dispensing patch unit can be carried out by one or more buttons/switches (1011) located on the dispensing patch unit (1010). The components of dispensing patch unit (1010) can be accommodated in one housing or in two housings. In the two housings configuration shown in FIG.2, the patch unit comprises reusable (1) and disposable (2) part as shown in co-owned, co-pending U.S. Patent Application No. 11/397,115 and International Patent Application No. PCT/IL2009/000388, published as WO 2009/125398.

In some arrangements, the remote control unit (1008) may contain a bolus delivery pattern selection feature (2000), a programmable processor (2010), a memory (2020), an input means (2030), e.g. buttons, switches, touch-screen, a display (2040) and other indication means (not shown) such as audible and vibration means (e.g. vibrator). The input means (2030) can preferably be provided for a bolus delivery pattern selection feature (2000) and for dispensing patch unit (1010) programming. The bolus delivery pattern selection feature can be a set of machine instructions for the programmable processor which are presented as machine-readable medium.

In some arrangements, the bolus delivery pattern selection feature can provide the capability for selection and/or recommendation of a bolus delivery pattern from a plurality of preprogrammed bolus delivery patterns stored in the memory (2020) and offered by the bolus delivery pattern selection feature. The available delivery patterns may be preprogrammed according to required bolus dose and at least one nutritional characteristic of a contemplated meal, e.g. according to GI.

According to some arrangements, the bolus delivery pattern selection feature can be located not only in the remote control unit, but may also be located (in place of or in addition to being provided in the remote control unit) in the reusable part of the dispensing patch unit (for example).

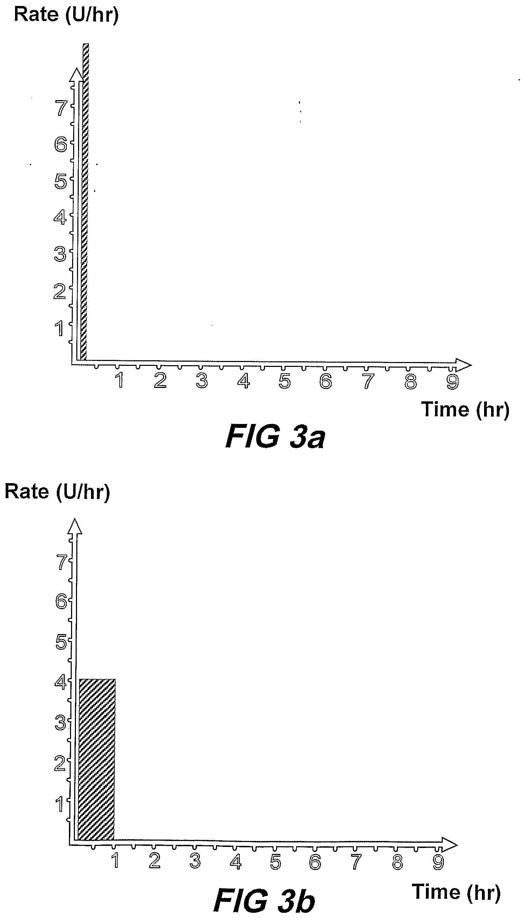

FIGs. 3a-e illustrate a display flow of displays, also referred to as graphical representations, of exemplary pre-programmed bolus delivery patterns, which can be suggested to the user by the device for selection. In some arrangements, the user can select from this plurality of preprogrammed patterns a particular delivery pattern which he/she finds suitable for balancing a contemplated meal. The vertical axis ("Rate") represents the rate of insulin delivery in Units of insulin per hour ("U/hr") for example, and the horizontal axis ("Time") represents the time during which insulin can be delivered, e.g. in hours ("hr"). The area under the curve/graph (referred-to also as "AUC") therefore equals product of the delivery Rate and the Time which results in the amount of bolus (in Units of insulin) being delivered during this Time. For the sake of illustration, the AUC associated with different delivery patterns corresponds to the same bolus dose in each of the illustrated delivery patterns depicted in FIGs. 3a-3e.

For example, in FIG. 3a, an exemplary delivery pattern is depicted where the entire bolus dose is delivered as rapidly as possible. This pattern may be suitable for a meal that is rapidly absorbed, e.g., a meal that is attributed as having a high GI. An example of such meal is cornflakes or fruit.

FIG. 3b depicts an exemplary delivery pattern in which the bolus dose is delivered evenly over a period of one hour. This delivery pattern may be suitable for a meal that is relatively slowly absorbed, e.g., a meal of a low GI or high in fat, such as pizza. This pattern may also be suitable for users with delayed gastric emptying due to gastroparesis, for example. This delivery pattern may also be appropriate for a meal to be eaten over a long period of time.

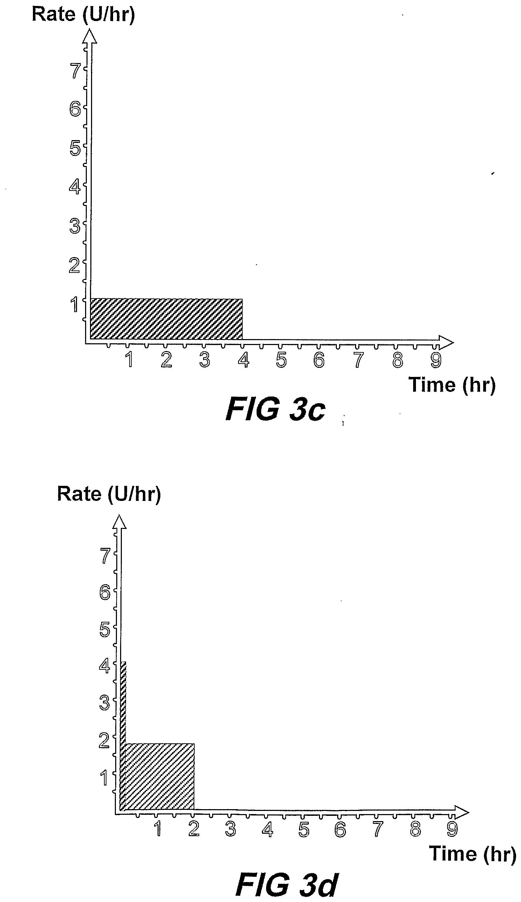

FIG. 3c depicts an exemplary delivery pattern in which the bolus dose is delivered evenly over a period of 4 hours (for example). This may be suitable for a meal that is very slowly absorbed e.g., a meal of a very low GI or very high in fat, such as steak. This delivery pattern may also be suitable for users with severe gastroparesis.

FIG. 3d depicts an exemplary delivery pattern in which a portion of the bolus dose is delivered immediately and the remaining bolus dose is delivered evenly, for example, over a prolonged period of time. In the depicted pattern, the immediate portion comprises, for example, 60% of the total bolus dose, and the remaining 40% are delivered over the following 2 hours (for example). Such a delivery pattern may be suitable for a meal that comprises both rapidly (meal with high GI) and slowly (meal with intermediate GI) absorbed carbohydrates, such as fruit and pasta. Such a pattern is also suitable when correction of elevated blood glucose value is needed before consuming a meal having an intermediate glycemic index. To that end, the insulin needed for correction of blood glucose is accounted for in the immediately delivered portion of the bolus dose and the slowly absorbed meal is accounted for in the bolus dose delivered during relatively long time interval. For example, if a user is planning to eat a meal having an intermediate GI and he\she has a high current blood glucose level, than the following two bolus delivery patterns are typically required:
- a correction bolus, which preferably delivered immediately, to bring the high BG level to a target value, and
- an extended bolus to balance the meal with an intermediate GI.

For example, a pattern such as depicted in FIG. 3d may be suitable to account for both bolus delivery patterns.

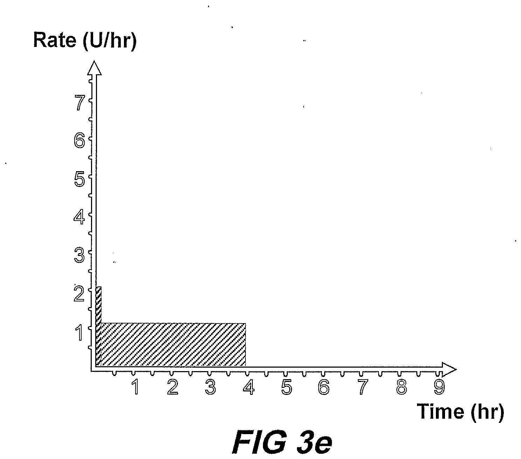

FIG. 3e depicts another exemplary bolus delivery pattern in which a portion of the bolus dose is delivered immediately and the remaining dose is delivered evenly over a prolonged period of time, for example. In the depicted pattern, the immediate portion comprises 60% of the total dose, for example, and the remaining 40% is delivered over the following 4 hours (for example. Such a delivery pattern may be suitable for a meal that comprises both rapidly (high GI) and very slowly (low GI) absorbed carbohydrates, such as fruit and pork ribs. Such a pattern is also suitable when correction of elevated blood glucose value is needed before a meal having low glycemic index.

In one arrangement, the most suitable preprogrammed delivery pattern can be recommended from a plurality of stored delivery patterns based on a user's input of a type of meal to be consumed (e.g. burger meal including fries and soft drink). In another arrangement, the most suitable delivery pattern can be recommended based on the user's input of a GI of the meal type (e.g. meal of a high/low/intermediate glycemic index).

In some arrangements, the insulin can be delivered automatically according to a selected preprogrammed delivery pattern corresponding to a specific meal to be consumed. For example, when a user provides a selection out of the meals list (e.g. a "pizza meal") the insulin can automatically be administered according to a preprogrammed for this type of meal bolus delivery pattern. In another arrangement, an appropriate bolus delivery pattern can be recommended to a user and accepted or ignored by the user. In some arrangements, the meal list with types of meal can be presented alpha numerically. The meal list can also be presented graphically on a screen of the remote control or on a screen of the dispensing device/patch unit (e.g., on the reusable part).

In some arrangements, the most suitable preprogrammed delivery pattern can be recommended according to the user's input of the GI or indexes of a meal.

In some arrangements, the user can program his/her bolus delivery patterns at an initial setting of the device. In some arrangements, fresh/new preprogrammed delivery patterns may be added to the plurality of already stored patterns or some or all old delivery patterns may be deleted.

According to one arrangement, the user may determine the number of delivery patterns that graphically and/or numerically be presented to him/her for selection of the delivery pattern, which is most appropriate for a specific meal.

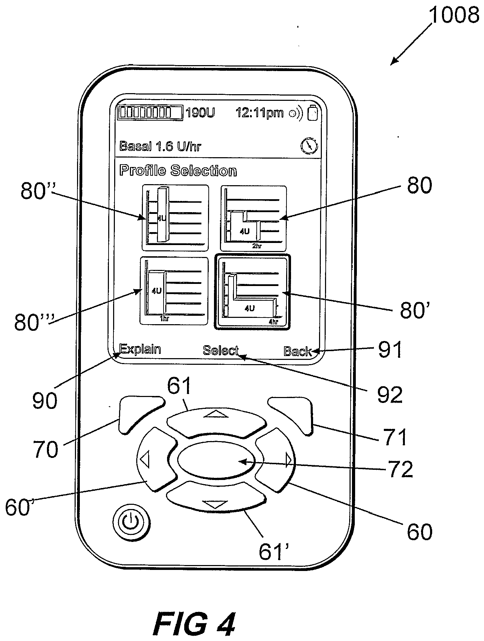

FIG. 4 shows a remote control unit (1008) that can be provided with a bolus delivery pattern selection feature. For example, the remote control unit may be provided with a display means, e.g. LCD screen, for displaying various icons/graphics associated with selection of the bolus delivery pattern, in particular, available for selection preprogrammed bolus delivery patterns. As seen in an example in FIG. 4, for example, the screen displays a plurality of icons (80, 80', 80", 80'"), wherein each icon graphically displays certain preprogrammed bolus delivery pattern.

In some arrangements, the displayed graphical representations of bolus delivery patterns can comprise one or more recommended bolus delivery patterns. Recommended bolus delivery pattern can, for example, be determined based on the information received from the user. Moreover, the recommended graphical representation can be highlighted, colored, framed, magnified, bolded, displayed in different font, pointed to by another object (e.g. an arrow that points to a recommended pattern). The recommended pattern can also be marked by using sound. For example, selection of the recommended representation can play a distinct sound, pitch or duration (e.g., a melody, a sound clip of a popular song, and the like). In some implementations, selection of the recommended pattern can cause the drug delivery device to vibrate a preset number of times. The preset number of times can be different for recommended patterns and for other patterns.

In some arrangements, the remote control unit may be provided with navigation buttons to enable the user to navigate between the icons using horizontal (60, 60') and vertical (61, 61') navigation buttons. By virtue of this provision, the user can select the icon associated with the bolus delivery pattern that he/she finds to be the most suitable for balancing the intake.

In some arrangements, the control unit may also be provided with a central key/button (72) (hereinafter referred to as a "soft" key) by pressing of which the user completes the selection. For example, the AUC of all displayed patterns (shown at icons 80, 80', 80", 80'") is generally identical and represent the required bolus dose to be delivered (in the given example the total bolus dose is equal to 4 units).

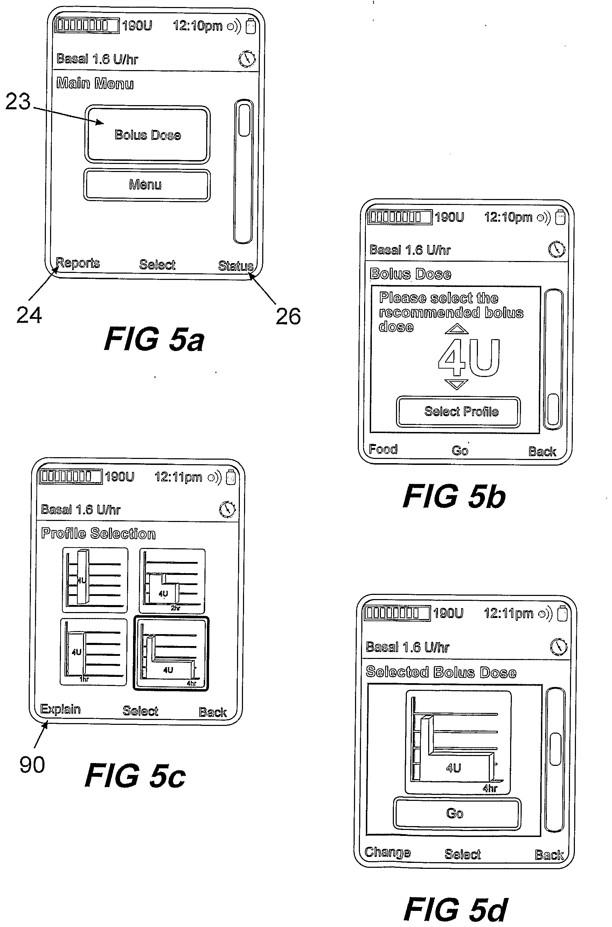

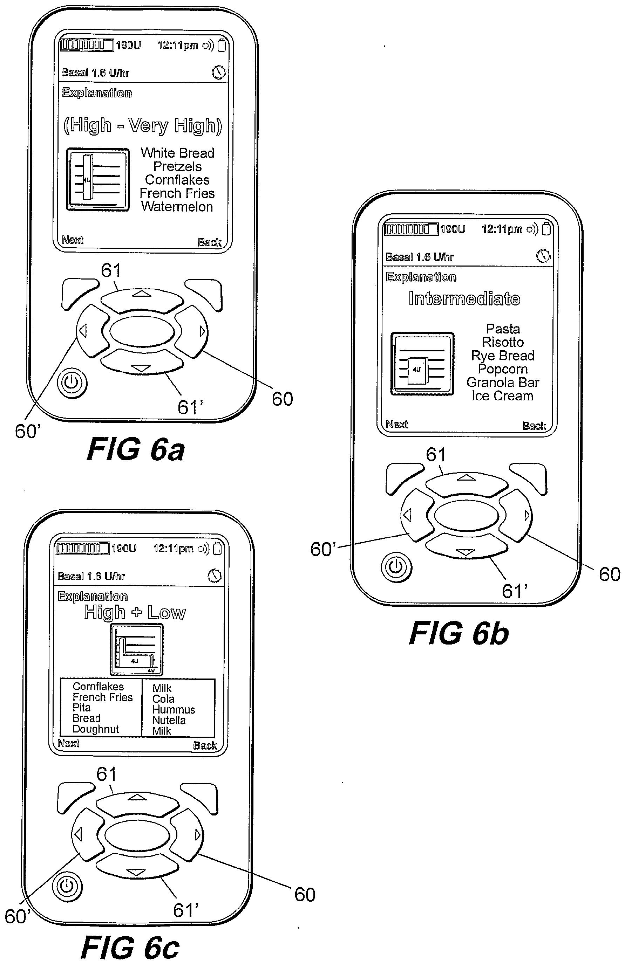

In some arrangements, the remote control unit is provided with additional soft keys, which include left soft key (70) and right soft key (71), while the display window is provided with additional icons (90), (91) and (92) which respectively display functions like, for example, "Explain", "Back" and "Select". By pressing of left, right and central soft keys, the user can execute the desired function displayed by the icons (90, 91, 92). For example, upon pressing the left soft key (70), the "Explain" icon (90) will be executed resulting in the window shown in detail in FIG 6. Upon pressing the right soft key (71), the "Back" icon (91) will be executed, allowing the user to go back to the previous display/window. Upon pressing the central soft key (72), the "Select" icon (92) will be executed, enabling the user to select the highlighted icon associated with the desired bolus delivery profile (e.g. 80' in FIG. 4). This selection allows the user to proceed to the next display, as shown in FIG. 5.

FIGs. 5a-d provide examples of screen displays of an exemplary user interface provided by the bolus delivery pattern selection feature. This display, for example, enables setting the bolus dose and selection of the bolus delivery pattern selection features. One of skill in the art will appreciate that the user interface may be displayed not only on a screen of the remote control unit, but also on a screen on the dispensing patch unit/device, or on both screens. This interface, on either component, can be provided with a plurality of navigation windows for data input.

For example, FIG. 5a shows an example of a main window of the bolus delivery pattern selection feature. If the user presses the central soft key (72) to select "Bolus Dose" icon (23), then the window shown in FIG. 5b can be displayed, for example. Alternatively, a window for downloading last administered bolus doses data may be displayed by selecting the "Reports" icon (24), for example. Upon selecting the "Status" icon (26), the user may display data associated with currently delivered boluses (for example).

FIG. 5b shows an example of a window which can be used for selection of a bolus dose. According to some arrangements, the bolus dose can be recommended by the device itself based on personal parameters (e.g. CIR, IS, current BG, target BG) associated with a specific user. The input of the bolus dose has been described, for example, in U.S. Patent Application No. 12/051,400 published as US 2008/0234663 and assigned to Medingo Ltd. Upon inputting the bolus dose and selecting the icon "Select profile" the bolus profile selection feature generates plurality of preprogrammed bolus delivery profiles, which will be displayed in the next window.

According to some arrangements, the most prevalent and/or averaged bolus delivery profile, which has been selected during a specific time interval, can be presented at this window (Fig. 5b) as well. This most prevalent/averaged delivery pattern can be presented, for example, as a first preferable choice ("default") instead of plurality of bolus delivery profiles. This feature may be especially beneficial for users with routine daily intakes.

FIG. 5c shows an example of a window, which displays a plurality of graphically represented bolus delivery patterns that can be suggested by the bolus delivery profile selection feature upon selecting the icon "Select profile". In some arrangements, one of the displayed patterns can be selected by the user. For example, the user can navigate between the displayed patterns and select the profile he/she finds the most suitable to balance the intake. In some arrangements, the "Explain" icon (90), for example, can also be provided. Selecting this icon by means of a soft key can enable the user with a short, general explanation of the meal content and its relevance to each bolus delivery pattern, as have been described above in connection with FIG. 3.

FIG. 5d shows one example of a user interface that represents the selected bolus delivery pattern. According to some arrangements, the user interface may illustrate a graphical representation of the selected pattern. This user interface may also comprise a "Go" icon, as well as "Change", "Select", and "Back" icons. For example, selecting the "Go" icon can indicate that the user wants the device to start administering the bolus (e.g. the insulin bolus) according to the graphically represented pattern.

According to some arrangements, for example, and as depicted in FIG.5d, the user has selected a profile shown in the low right corner of the window shown in FIG.5c and now upon selecting the "Go" icon, the bolus dose of four units will be delivered at a delivery rate corresponding to the selected bolus delivery profile.

FIGs. 6a-c illustrate examples of three windows displaying a short, general explanation of the meal content and its relevance to each bolus pattern. In some arrangements, for example, the user interface windows on FIGs. 6a-c can be reached by selecting the "Explain" icon (90) upon pressing the soft key, as provided in FIG. 4.

FIG. 6a, for example, illustrates an example window that contains a brief explanation of the food type (high-very high GI). In the same window, a bolus delivery profile may also be graphically presented, which is suitable for this food type and also are provided examples of other food product associated with this type (e.g. white bread, pretzels). According to the bolus delivery pattern shown in this figure, the bolus dose will be delivered immediately, for example.

FIG. 6b illustrates an exemplary window that briefly explains the food type (intermediate GI) suitably balanced by alternative, graphically presented delivery profile, and also provides examples of food products associated with this food type (e.g. pasta, pizza), for example. According to this delivery pattern, the bolus dose should be delivered evenly over a period of 2 hours for example.

FIG. 6c illustrates an exemplary window that, for example, briefly explains the food type (a combination of low GI food type and high-very high GI food type) which may suitably be balanced, for example, by another graphically presented delivery profile, which is recommended in this case. The window may also provide examples of food products associated with this food type (e.g. milk and cornflakes, pita and hummus, etc.). According to the recommended delivery pattern, a portion of the bolus dose should be delivered immediately and the remaining dose should be delivered evenly over 4 hours, for example. This bolus pattern can also be helpful when a correction bolus is needed (in the case of a high blood glucose level) and a meal of a low GI content is contemplated for intake, for example. In some arrangements, the immediately delivery bolus phase can follow the bolus delivered evenly over time, for example.

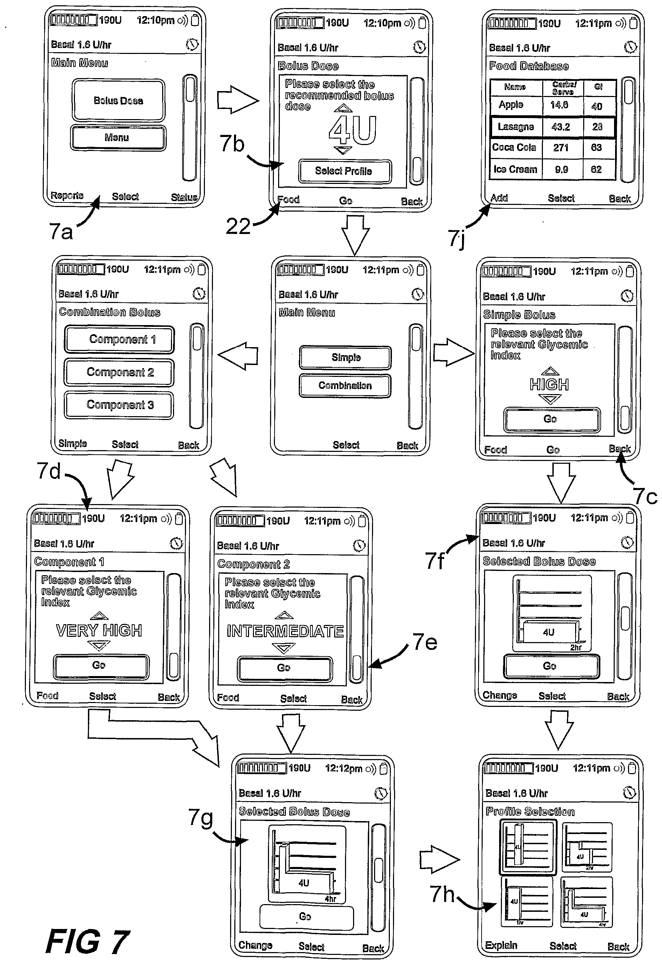

FIGs. 7a-j illustrate other examples of user interfaces that can be provided by the bolus delivery pattern selection feature. Specifically, the use interface designated in 7a, for example, provides an example of a main window of the bolus delivery pattern selection feature. In some arrangements, if the user presses on the soft key to select the icon "Bolus Dose", then the window 7b can be displayed. Further icons may be selected by means of additional soft keys.

The window 7b is an example of a window for selection of a bolus dose. According to some arrangements, the bolus dose can be recommended by the device upon inputting parameters associated with the user. According to one arrangement, the most prevalent and/or averaged bolus delivery profile selected during a specific time interval can be presented at this window, for example. Thus, selection of the appropriate bolus delivery pattern can be accomplished even prior to loading the GI of the intake. In some arrangements, the most prevalent and/or averaged delivery pattern can be presented as a first preferable choice ("default"), for example. This feature may be especially beneficial for users with routine daily intakes.

The window illustrated in Fig. 7c provides an example of a window for inputting GI of the intake of a single meal. The GI in the example can be selected as a qualitative parameter - e.g. low, intermediate, high, and very high GI. The qualitative presentation of the GI can be translated to numerical values according to, for example, the following schedule:
Low GI **→** <30
Intermediate GI **→** 30-54
High GI **→** 55-79
Very high GI **→** 80-100

The cutoff values between ranges may be different and the number of GI ranges may be more or less than in the given exemplary schedule, for example. If a meal comprises different foods having more than one GI (for example pizza and cake for dessert), a "combined GI" may be selected and the GI's of the different meal components are entered one after the other in different consecutive windows, designated respectively by respective numerals 7d, 7e. For example, the window 7d relates to the first component of the "combined" meal and window 7e relates to the second component.

Windows 7f-7g provide examples of windows which graphically display recommended bolus delivery patterns according to the GI of the meal. Specifically, the window in 7f, for example, corresponds to a "simple" meal comprising a single component and the window in 7g corresponds to a "combined" meal comprising two components with two different GIs, as shown in windows 7d-e. The bolus delivery pattern selection feature graphically presents delivery patterns that can be suitable for meals containing combined glycemic indexes of, for example, high GI + Low GI or high GI + intermediate GI. In one variation, the user can select the appropriate bolus delivery pattern by accepting the recommended delivery pattern or select a different pattern by navigating between the different patterns, for example between those shown in the window shown in Fig. 7h.

In some arrangements, the user may also display a database with parameters associated with different foods via the windows designated by numerals 7b-e by pressing the soft key and selecting the "food" icon (22), for example. Fig. 7j illustrates is an example of a window displaying such a food database providing the carb load and a corresponding GI value for different food products.

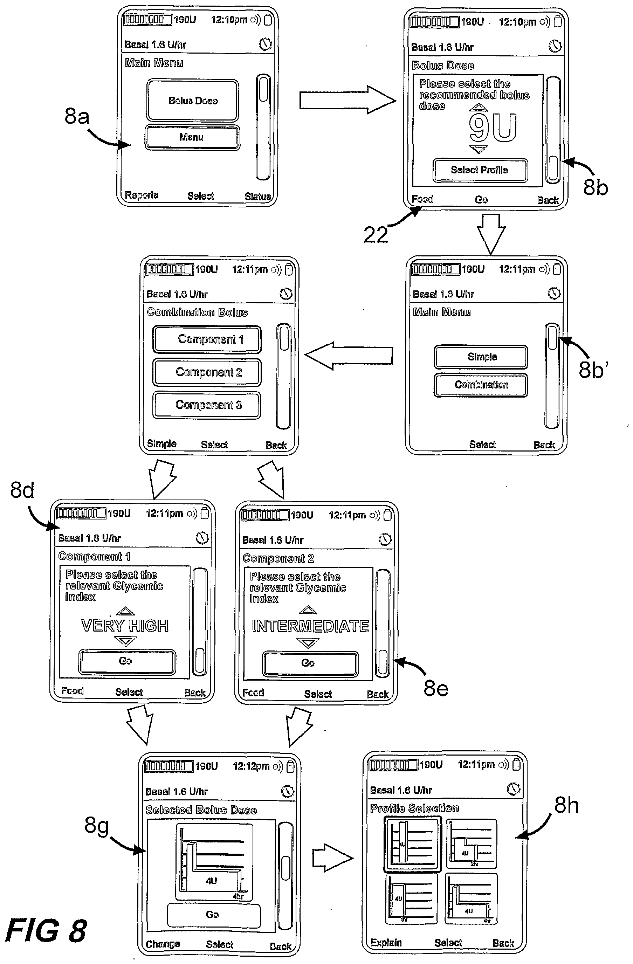

FIG. 8 provides another example of windows with a user interface for selection of a bolus delivery pattern for a specific meal comprising pasta and lemonade. In one implementation, a bolus dose of 9 IU is selected in the window 8b to balance the contemplated meal. In this exemplary arrangement, it is supposed that the meal includes 90 grams of carbohydrates, and a user carbohydrate to insulin ratio is 10 grams/units -- in such a case, no correction bolus is needed. In the window 8b', the user selects a "combination" icon for the meal, for example. The GI of the first component - lemonade, as shown in window 8d, is very high. The GI of the second component- pasta, as shown in window 8e, is intermediate.

The window shown in Fig. 8g, which is reached after navigating through the previous windows (for example), provides a graphical display of certain recommended bolus delivery patterns corresponding to the GI of the meal. The recommended bolus delivery pattern in this scenario, for example, illustrates that a portion of the bolus dose can be delivered immediately to account for the lemonade and the remaining dose can be delivered evenly over a prolonged period of time to account for the pasta meal.

In some arrangements, the user may select the recommended delivery pattern as shown in the window illustrated in Fig. 8g, or select a different pattern by navigating to the window shown in Fig. 8h, which displays a plurality of preprogrammed patterns available for selection, for example.

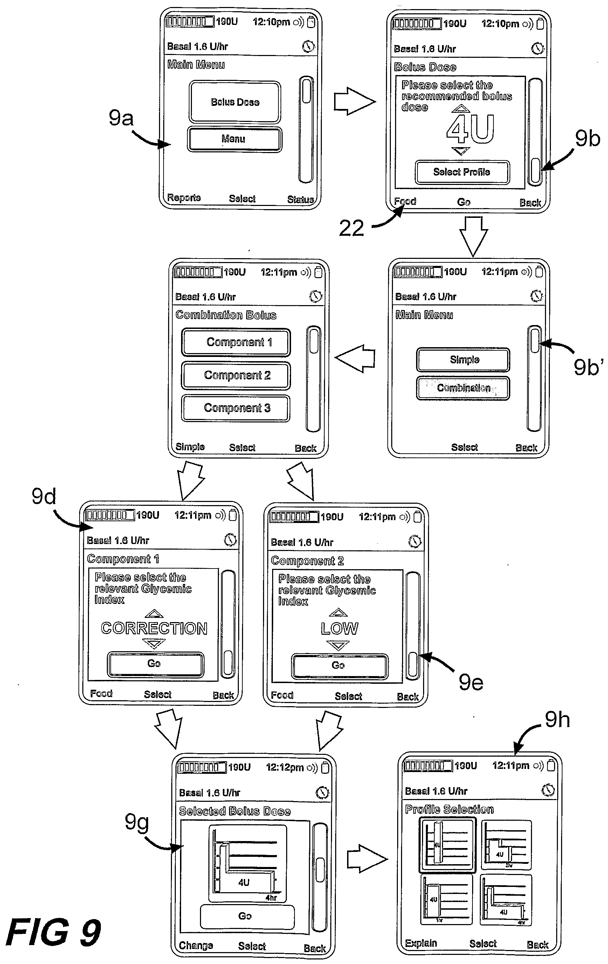

FIG. 9 provides an additional example of a user interface for selection of a bolus delivery pattern. This interface can be used by a user who has a high blood glucose value before eating, and intends to eat a salad which has a low GI.

For example, a bolus dose of 4 IU can be selected in the window designated by Fig. 9b. This bolus dose, for example, is required to balance a meal which includes 40grams of carbohydrates, for a user whose carbohydrate to insulin ration is 10grams/units, and no correction bolus is needed. Administering of the selected bolus dose can bring the user's blood glucose level to the target blood glucose level (by administering correction bolus) and then to balance the contemplated salad meal. In the window of 9b', the user can select a "combination" icon for the meal. By inputting the first component of the combination meal as shown in the window of Fig. 9d, the "correction" portion of the bolus will be defined. The GI of the second component - salad - is low and it is inputted as shown in the window of Fig. 9e.

The window of Fig. 9g shows a graphical display of the recommended bolus delivery pattern corresponding to the data inputted through previous windows. According to this pattern, a portion of the bolus dose is delivered immediately to account for the "correction bolus" and the remaining dose is delivered evenly over a prolonged period of time to account for the salad, for example. The user may select the recommended pattern or select a different pattern by navigating to the window in Fig. 9h, which displays a plurality of preprogrammed patterns available for selection, for example.

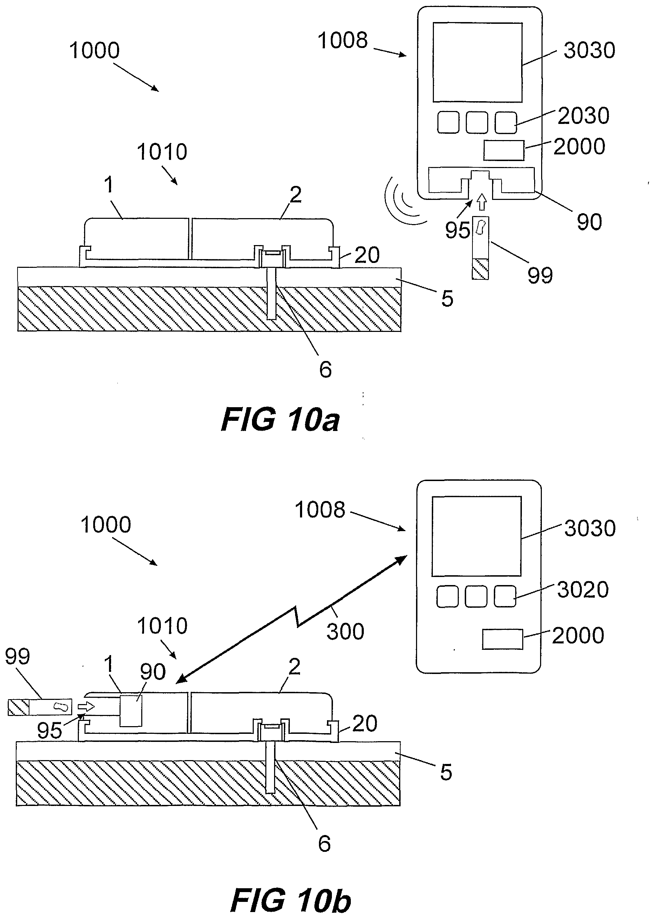

FIGs. 10a-c provide alternate arrangements of a drug delivery system/device, e.g. an insulin infusion system/device. In these arrangements, the device may comprise an insulin dispensing unit, a remote control unit that can be provided with a bolus delivery pattern selection feature (2000), and a means for blood glucose monitoring (e.g. "glucometer"), for example.

For example, FIG. 10a shows a glucometer (90) located in the remote control unit (1008) of the device (which may also be provided in the dispensing unit). The glucometer (90) comprises an opening (95) for receiving of a test strip (99). The user extracts blood from the body, places a blood drop on the test strip (99) and inserts the strip (99) into the opening (95). The glucose readings are displayed on a screen (3030) of the remote control unit (1008), for example.

FIG. 10b shows an example of an arrangement in which the dispensing unit is configured as a patch unit having reusable and disposable parts, for example. The dispensing unit has a glucometer (90) located in the reusable part (1) of the patch unit (1010), though it may also be possible to locate the glucometer in any portion of the patch unit, and/or the remote unit.

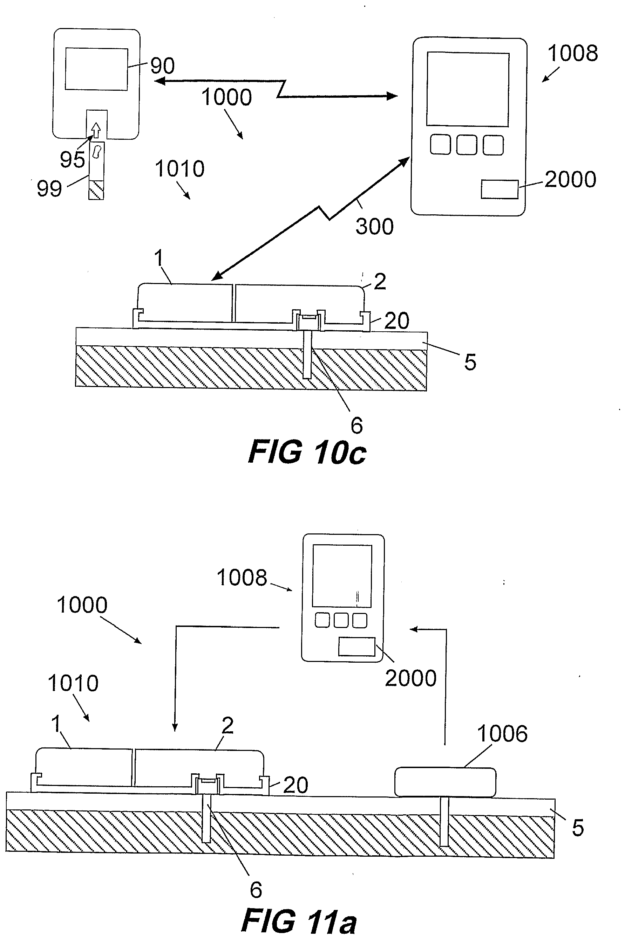

FIG. 10c shows an example of an arrangement in which glucose readings are directly or remotely received from a glucometer (90), for example, which in this arrangement constitutes an independent glucometer.

FIGs. 11a-b show alternate arrangements of a drug delivery system/device, e.g. an insulin infusion system/device comprising an insulin dispensing unit, a remote control unit that can be provided with a bolus delivery pattern selection feature (2000), and a continuous subcutaneous glucose monitor (1006).

Specifically, FIG. 11a shows an example of an arrangement in which the current blood glucose concentration can be received from an independent continuous subcutaneous glucose sensing apparatus (1006).

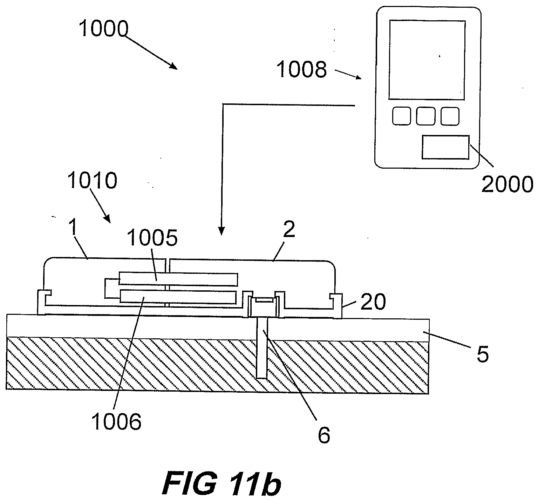

FIG. 11b shows an example of an arrangement in which the continuous subcutaneous glucose sensing apparatus (1006) is located in the dispensing patch unit (1010) of the insulin infusion device.

As disclosed in our previous PCT application PCT/IL07/000163, published as WO 2007/093981, the dispensing patch unit (1010) is provided with an insulin dispensing apparatus (1005) and glucose sensing apparatus (1006). These components constitute, in the illustrated arrangement, a common insulin infusion device, and they may share a single cannula (6) for both dispensing insulin and sensing glucose. However, in an alternative arrangement the sensing apparatus and the dispensing apparatus may have separate cannulae that penetrate the skin (5) and reside in the subcutaneous tissue.

In another arrangement, a drug delivery system/device can be configured to operate as a semi-closed loop system. In a semi-closed loop system, the feedback and control between sensor's measurements of analyte concentration and associated drug administration can be partially automatic. For example, the release of drug at a basal rate can be automatically controlled by the processor based on analytes measurements, while the release of bolus doses of the drug can still be effected according to the user's input. In the case when the drug is insulin and the analyte is glucose the drug can automatically be dispensed according to continuous monitoring of glucose levels and according additional pre-meal bolus user inputs (semi-closed loop). The bolus delivery pattern selection feature (2000) can be used for bolus inputs in such semi-closed loop system, for example.

Various implementations of the bolus delivery pattern selection feature described herein may be realized in digital electronic circuitry, integrated circuitry, specially designed ASICs (application specific integrated circuits), computer hardware, firmware, software, and/or combinations thereof. These various implementations may include implementation in one or more computer programs that are executable and/or interpretable on a programmable system including at least one programmable processor, which may be special or general purpose, coupled to receive data and instructions from, and to transmit data and instructions to, a storage system, at least one input device, and at least one output device.

These computer programs (also known as programs, software, software applications or code) include machine instructions for a programmable processor, for example, and may be implemented in a high-level procedural and/or object-oriented programming language, and/or in assembly/machine language. As used herein, the term "machine-readable medium" refers to any computer program product, apparatus and/or device (e.g., magnetic discs, optical disks, memory, Programmable Logic Devices (PLDs)) used to provide machine instructions and/or data to a programmable processor, including a machine-readable medium that receives machine instructions as a machine-readable signal. The term "machine-readable signal" refers to any signal used to provide machine instructions and/or data to a programmable processor.

To provide for interaction with a user, the subject matter described herein may be implemented on a computer having a display device (e.g., a CRT (cathode ray tube) or LCD (liquid crystal display) monitor) for displaying information to the user and a keyboard and a pointing device (e.g., a mouse or a trackball) by which the user may provide input to the computer. For example, this program can be stored, executed and operated by the dispensing unit, remote control, PC, laptop or personal data assistant ("PDA"). Other kinds of devices may be used to provide for interaction with a user as well; for example, feedback provided to the user may be any form of sensory feedback (e.g., visual feedback, auditory feedback, or tactile feedback); and input from the user may be received in any form, including acoustic, speech, or tactile input, such as that after selecting a desired fluid delivery pattern (e.g., pattern no. 3), the remote control unit will sound an auditory feedback: "Pattern no. 3 has been selected. Please confirm."

Certain arrangements of the subject matter described herein may be implemented in a computing system and/or devices that includes a back-end component (e.g., as a data server), or that includes a middleware component (e.g., an application server), or that includes a front-end component (e.g., a client computer having a graphical user interface or a Web browser through which a user may interact with an implementation of the subject matter described herein), or any combination of such back-end, middleware, or front-end components. The components of the system may be interconnected by any form or medium of digital data communication (e.g., a communication network). Examples of communication networks include a local area network ("LAN"), a wide area network ("WAN"), and the Internet.

The computing system according to some such arrangements described above may include clients and servers. A client and server are generally remote from each other and typically interact through a communication network. The relationship of client and server arises by virtue of computer programs running on the respective computers and having a client-server relationship to each other. For example, a patient that does not have his remote control unit "at arm's length", can administer and control a bolus dose administration via the internet. Another implementation refers to a physician that is located far from the patient and device, but still able to monitor, operate and receive data from the device via the internet or a data server, e.g., a U.S. based physician can communicate with the device and patient which are situated overseas.

Preferable arrangements implement the bolus selection feature via software operated on a processor contained in a remote control device of an insulin dispensing system and/or a processor contained in a insulin dispensing device being party of an insulin dispensing system.

Although a few variations have been described in detail above, other modifications are possible. For example, the logic flow depicted in the accompanying figures and described herein does not require the particular order shown, or sequential order, to achieve desirable results. Other implementations may be within the scope of the following claims.

## Claims

1. A drug delivery device (1000) comprising:
a first module adapted for receiving a drug bolus to be delivered;
a memory (2020), the memory (2020) being adapted for storing a plurality of pre-programmed bolus delivery patterns, with each bolus delivery pattern defining the rate or rates at which the drug bolus is administered to the user;
a dispensing unit (1010) adapted for delivering the received drug bolus to the user;
a screen (2040) adapted for providing to a user a graphical representation (80,80',80",80"') of each of the plurality pre-programmed bolus delivery patterns in accordance with the received drug bolus to be delivered; and
a second module adapted for receiving a user selection identifying the graphical representation (80,80',80",80'") of one of the plurality of pre-programmed bolus delivery patterns;
the dispensing unit (1010) being configured to deliver the received drug bolus to the user at a drug delivery rate in accordance with the pre-programmed bolus delivery pattern of the graphical representation selected by the user;
**characterized in that**: the plurality of pre-programmed bolus delivery patterns are adapted to meals containing different foods having more than one glycemic index; and at least one of the plurality of pre-programmed bolus delivery patterns is recommended to the user for selection based upon inputted parameters associated with the user which are in accordance with nutritional characteristics of a contemplated meal comprising different foods having more than one glycemic index, with the glycemic index of the different foods of the contemplated meal being entered one after the other.

2. The drug delivery device (1000) of claim 1, wherein the drug comprises insulin and the dispensing unit (1010) comprises an insulin pump.

3. The drug delivery device (1000) of claim 1, wherein the graphical representation (80') of the recommended bolus delivery pattern is visually distinct from the graphical representation (80,80",80"') of at least one other bolus delivery pattern.

4. The drug delivery device (1000) of claim 1, wherein at least one of the plurality graphical representations (80,80') corresponds to a bolus delivery pattern comprising at least two different delivery rates.

5. The drug delivery device (1000) of claim 1, wherein at least one of the plurality graphical representations corresponds to a bolus delivery pattern comprising correction of blood glucose value and counteracting a contemplated meal.

6. The drug delivery device (1000) of claim 1, further comprising a user interface adapted for modification of the bolus delivery duration corresponding to one of the plurality pre-programmed bolus delivery patterns.

7. The drug delivery device (1000) of claim 1, wherein the memory (2020) is further adapted for storing one or more of the selected bolus delivery patterns.

8. The drug delivery device (1000) of claim 7, wherein the memory (2020) is further adapted for storing a point in time of each of the one or more selected bolus delivery patterns.

9. The drug delivery device (1000) of claim 7, wherein at least one of the plurality graphical representations of the plurality pre-programmed bolus delivery patterns corresponds to one of the one or more stored selected bolus delivery patterns.

10. The drug delivery device (1000) of claim 8, wherein at least one of the plurality graphical representations is recommended to the user for selection, the recommended bolus delivery pattern is selected from the one or more stored selected bolus delivery patterns in accordance with the stored point of time.

11. The drug delivery device (1000) of claim 2, wherein each graphical representation has a vertical axis representing the rate of insulin delivery and a horizontal axis representing the time during which insulin can be delivered.

12. A bolus delivery pattern selection method for selecting a bolus delivery pattern for delivering a drug bolus to a user by a drug delivery device, the method comprising:
providing a drug delivery system (1000), including a drug infusion device (1010) and optionally including a remote control device (1008) to send and receive information and/or instructions to the drug infusion device, wherein at least one of the drug delivery device and the remote control device includes a display;
storing a plurality of pre-programmed bolus delivery patterns in a memory (2020) of at least one of the remote control unit and the drug infusion device, with each bolus delivery pattern defining the rate or rates at which the drug bolus is administered to the user;
providing to a user, via the display, a graphical representation (80,80',80",80"') of each of the plurality of pre-programmed bolus delivery patterns, in accordance with the received amount of drug bolus;
receiving a user selection via at least one of the remote control device and the drug infusion device, identifying one of the plurality of pre-programmed bolus delivery patterns; and
determining a drug delivery rate for delivering the drug bolus to the user in accordance with the bolus delivery pattern identified by the user selection;
**characterized in that**: the plurality of programmed pre-programmed bolus delivery patterns are adapted to meals containing different foods having more than one glycemic index; and at least one of the plurality pre-programmed bolus delivery patterns is recommended to the user for selection based upon inputted parameters associated with the user which are in accordance with nutritional characteristics of a contemplated meal comprising different foods having more than one glycemic index, with the glycemic index of the different foods of the contemplated meal being entered one after the other.

## Patentansprüche

1. Arzneimittelabgabevorrichtung (1000), umfassend:
ein erstes Modul, das zum Empfangen eines abzugebenden Arzneimittelbolus angepasst ist;
einen Speicher (2020), wobei der Speicher (2020) angepasst ist, um eine Vielzahl vorprogammierter Bolusabgabemuster zu speichern, wobei jedes Bolusabgabemuster die Rate oder Raten definiert, bei der/denen der Arzneimittelbolus dem Nutzer verabreicht wird;
eine Abgabeeinheit (1010), die zum Abgeben des empfangenen Arzneimittelbolus an den Nutzer angepasst ist;
einen Bildschirm (2040), der angepasst ist, um einem Nutzer eine graphische Darstellung (80, 80', 80", 80"') jedes der Vielzahl vorprogrammierter Bolusabgabemuster gemäß dem empfangenen abzugebenden Arzneimittelbolus bereitzustellen;
und ein zweites Modul, das angepasst ist, um eine Nutzerauswahl zu empfangen, die die graphische Darstellung (80, 80', 80", 80'") eines der Vielzahl vorprogrammierter Bolusabgabemuster identifiziert;
wobei die Abgabeeinheit (1010) konfiguriert ist, um den empfangenen Arzneimittelbolus bei einer Arzneimittelabgaberate gemäß dem vorprogrammierten Bolusabgabemuster der graphischen Darstellung, die vom Nutzer ausgewählt wurde, an den Nutzer abzugeben;
**dadurch gekennzeichnet, dass**: die Vielzahl vorprogrammierter Bolusabgabemuster an Mahlzeiten angepasst ist, die unterschiedliche Nahrungsmittel mit mehr als einem glykämischen Index enthalten; und mindestens eines der Vielzahl vorprogrammierter Bolusabgabemuster dem Nutzer auf der Basis eingegebener Parameter zur Auswahl empfohlen wird, die dem Nutzer zugeordnet sind und Nährwerteigenschaften einer beabsichtigten Mahlzeit entsprechen, die unterschiedliche Nahrungsmittel mit mehr als einem glykämischen Index enthält, wobei der glykämische Index der unterschiedlichen Nahrungsmittel der beabsichtigten Mahlzeit jeweils nacheinander eingegeben wird.

2. Arzneimittelabgabevorrichtung (1000) nach Anspruch 1, wobei das Arzneimittel Insulin umfasst und die Abgabeeinheit (1010) eine Insulinpumpe umfasst.

3. Arzneimittelabgabevorrichtung (1000) nach Anspruch 1, wobei sich die graphische Darstellung (80') des empfohlenen Bolusabgabemusters visuell von der graphischen Darstellung (80, 80", 80'") mindestens eines anderen Bolusabgabemusters unterscheidet.

4. Arzneimittelabgabevorrichtung (1000) nach Anspruch 1, wobei mindestens eine der Vielzahl graphischer Darstellungen (80, 80') einem Bolusabgabemuster entspricht, das mindestens zwei unterschiedliche Abgaberaten umfasst.

5. Arzneimittelabgabevorrichtung (1000) nach Anspruch 1, wobei mindestens eine der Vielzahl graphischer Darstellungen einem Bolusabgabemuster entspricht, das eine Korrektur des Blutzuckerwerts und das Entgegenwirken einer beabsichtigten Mahlzeit umfasst.

6. Arzneimittelabgabevorrichtung (1000) nach Anspruch 1, ferner umfassend eine Nutzerschnittstelle, die zur Modifikation der Bolusabgabedauer entsprechend einem der Vielzahl vorprogrammierter Bolusabgabemuster angepasst ist.

7. Arzneimittelabgabevorrichtung (1000) nach Anspruch 1, wobei der Speicher (2020) ferner angepasst ist, um eines oder mehrere der ausgewählten Bolusabgabemuster zu speichern.

8. Arzneimittelabgabevorrichtung (1000) nach Anspruch 7, wobei der Speicher (2020) ferner angepasst ist, um einen Zeitpunkt jedes des einen oder der mehreren ausgewählten Bolusabgabemuster zu speichern.

9. Arzneimittelabgabevorrichtung (1000) nach Anspruch 7, wobei mindestens eine der Vielzahl graphischer Darstellungen der Vielzahl vorprogrammierter Bolusabgabemuster dem einen oder den mehreren gespeicherten ausgewählten Bolusabgabemustern entspricht.

10. Arzneimittelabgabevorrichtung (1000) nach Anspruch 8, wobei mindestens eine der Vielzahl graphischer Darstellungen dem Nutzer zur Auswahl empfohlen wird, das empfohlene Bolusabgabemuster gemäß dem gespeicherten Zeitpunkt aus dem einen oder den mehreren gespeicherten ausgewählten Bolusabgabemustern ausgewählt wird.

11. Arzneimittelabgabevorrichtung (1000) nach Anspruch 2, wobei jede graphische Darstellung eine vertikale Achse, die die Rate der Insulinabgabe darstellt, und eine horizontale Achse aufweist, die die Zeit darstellt, während derer Insulin abgegeben werden kann.

12. Bolusabgabemuster-Auswahlverfahren zum Auswählen eines Bolusabgabemusters zum Abgeben eines Arzneimittelbolus an einen Nutzer durch eine Arzneimittelabgabevorrichtung, wobei das Verfahren umfasst:
Bereitstellen eines Arzneimittelabgabesystems (1000), enthaltend eine Arzneimittelinfusionsvorrichtung (1010) und optional enthaltend eine Fernsteuervorrichtung (1008) zum Senden und Empfangen von Informationen und/oder Anweisungen an die Arzneimittelinfusionsvorrichtung, wobei mindestens eine der Arzneimittelabgabevorrichtung und der Fernsteuervorrichtung eine Anzeige enthält;
Speichern einer Vielzahl vorprogrammierter Bolusabgabemuster in einem Speicher (2020) mindestens einer der Fernsteuereinheit und der Arzneimittelinfusionsvorrichtung, wobei jedes Bolusabgabemuster die Rate oder Raten definiert, bei denen der Arzneimittelbolus an den Nutzer verabreicht wird;
Bereitstellen einem Nutzer, über die Anzeige, einer graphischen Darstellung (80, 80', 80", 80"') jedes der Vielzahl vorprogrammierter Bolusabgabemuster gemäß der empfangenen Menge des Arzneimittelbolus;
Empfangen einer Nutzerauswahl über mindestens eine der Fernsteuervorrichtung und der Arzneimittelinfusionsvorrichtung, Identifizieren eines der Vielzahl vorprogrammierter Bolusabgabemuster;
und Bestimmen einer Arzneimittelabgaberate zum Abgeben des Arzneimittelbolus an den Nutzer gemäß dem durch die Nutzerauswahl identifizierten Bolusabgabemuster;
**dadurch gekennzeichnet, dass**: die Vielzahl programmierter vorprogrammierter Bolusabgabemuster an Mahlzeiten angepasst ist, die unterschiedliche Nahrungsmittel mit mehr als einem glykämischen Index enthalten; und mindestens eines der Vielzahl vorprogrammierter Bolusabgabemuster dem Nutzer auf der Basis eingegebener Parameter zur Auswahl empfohlen wird, die dem Nutzer zugeordnet sind und Nährwerteigenschaften einer beabsichtigten Mahlzeit entsprechen, die unterschiedliche Nahrungsmittel mit mehr als einem glykämischen Index enthält, wobei der glykämische Index der unterschiedlichen Nahrungsmittel der beabsichtigten Mahlzeit jeweils nacheinander eingegeben wird.

## Revendications

1. Dispositif de délivrance de médicament (1000) comprenant :
un premier module adapté pour recevoir un bolus de médicament devant être délivré ;
une mémoire (2020), la mémoire (2020) étant adaptée pour stocker une pluralité de modèles de délivrance de bolus pré-programmés, avec chaque modèle de délivrance de bolus définissant le ou les débit(s) au(x)quel(s) le bolus de médicament est administré à l'utilisateur ;
une unité distributrice (1010) adaptée pour délivrer le bolus de médicament reçu à l'utilisateur ;
un écran (2040) adapté pour présenter à un utilisateur une représentation graphique (80, 80', 80", 80"') de chacun de la pluralité de modèles de délivrance de bolus pré-programmés en fonction du bolus de médicament reçu devant être délivré ;
et un deuxième module adapté pour recevoir une sélection d'utilisateur identifiant la représentation graphique (80, 80', 80", 80'") de l'une de la pluralité de modèles de délivrance de bolus pré-programmés ;
l'unité distributrice (1010) étant configurée pour délivrer le bolus de médicament reçu à l'utilisateur à un débit de délivrance de médicament en fonction du modèle de délivrance de bolus pré-programmé de la représentation graphique sélectionnée par l'utilisateur ;
**caractérisé en ce que** : la pluralité de modèles de délivrance de bolus pré-programmés sont adaptés à des repas contenant différents aliments possédant plus d'un indice glycémique ; et au moins l'un de la pluralité de modèles de délivrance de bolus pré-programmés est recommandé à l'utilisateur pour sélection sur la base de paramètres entrés associés à l'utilisateur qui sont en fonction de caractéristiques nutritionnelles d'un repas envisagé comprenant différents aliments possédant plus d'un indice glycémique, avec l'indice glycémique des différents aliments du repas envisagé étant entrés l'un après l'autre.

2. Dispositif de délivrance de médicament (1000) selon la revendication 1, dans lequel le médicament comprend de l'insuline et l'unité distributrice (1010) comprend une pompe à insuline.

3. Dispositif de délivrance de médicament (1000) selon la revendication 1, dans lequel la représentation graphique (80') du modèle de délivrance de bolus recommandé est distincte visuellement de la représentation graphique (80, 80", 80'") d'au moins un autre modèle de délivrance de bolus.

4. Dispositif de délivrance de médicament (1000) selon la revendication 1, dans lequel au moins l'une de la pluralité de représentations graphiques (80,80') correspond à un modèle de délivrance de bolus comprenant au moins deux débits de délivrance différents.

5. Dispositif de délivrance de médicament (1000) selon la revendication 1, dans lequel au moins l'une de la pluralité de représentations graphiques correspond à un modèle de délivrance de bolus comprenant une correction de valeur de glycémie et annihilant un repas envisagé.

6. Dispositif de délivrance de médicament (1000) selon la revendication 1, comprenant en outre une interface utilisateur adaptée pour la modification de la durée de délivrance du bolus correspondant à l'un de la pluralité de modèles de délivrance de bolus pré-programmés.

7. Dispositif de délivrance de médicament (1000) selon la revendication 1, dans lequel la mémoire (2020) est en outre adaptée pour stocker l'un ou plusieurs des modèles de délivrance de bolus sélectionnés.

8. Dispositif de délivrance de médicament (1000) selon la revendication 7, dans lequel la mémoire (2020) est en outre adaptée pour stocker un point temporel de chacun de l'un ou plusieurs des modèles de délivrance de bolus sélectionnés.

9. Dispositif de délivrance de médicament (1000) selon la revendication 7, dans lequel au moins l'une de la pluralité de représentations graphiques de la pluralité de modèles de délivrance de bolus pré-programmés correspond à l'un de l'un ou plusieurs modèles de délivrance de bolus sélectionnés stockés.

10. Dispositif de délivrance de médicament (1000) selon la revendication 8, dans lequel au moins l'une de la pluralité de représentations graphiques est recommandée à l'utilisateur pour sélection, le modèle de délivrance de bolus recommandé est sélectionné parmi l'un ou plusieurs modèles de délivrance de bolus sélectionnés stockés en fonction du point temporel stocké.

11. Dispositif de délivrance de médicament (1000) selon la revendication 2, dans lequel chaque représentation graphiques a un axe vertical représentant le débit de délivrance d'insuline et un axe horizontal représentant la durée pendant laquelle l'insuline peut être délivrée.

12. Procédé de sélection d'un modèle de délivrance de bolus permettant la sélection d'un modèle de délivrance de bolus destiné à délivrer un bolus de médicament à un utilisateur par un dispositif de délivrance de médicament, le procédé comprenant :
la fourniture d'un système de délivrance de médicament (1000), incluant un dispositif de perfusion de médicament (1010) et incluant en option un dispositif de commande à distance (1008) pour envoyer et recevoir des informations et/ou instructions au dispositif de perfusion de médicament, dans lequel au moins l'un du dispositif de délivrance de médicament et du dispositif de commande à distance inclut un affichage ;
le stockage d'une pluralité de modèles de délivrance de bolus pré-programmés dans une mémoire (2020) d'au moins l'un de l'unité de commande à distance et du dispositif de perfusion de médicament, avec chaque modèle de délivrance de bolus définissant le ou les débit(s) au(x)quel(s) le bolus de médicament est administré à l'utilisateur ;
la présentation à un utilisateur, via l'affichage, d'une représentation graphique (80, 80', 80", 80"') de chacun de la pluralité de modèles de délivrance de bolus pré-programmés, en fonction de la quantité reçue de bolus de médicament ;
la réception d'une sélection d'utilisateur via au moins l'un du dispositif de commande à distance et du dispositif de perfusion de médicament, l'identification de l'un de la pluralité de modèles de délivrance de bolus pré-programmés ;
et la détermination d'un débit de délivrance de médicament pour la délivrance du bolus de médicament à l'utilisateur en fonction du modèle de délivrance de bolus identifié par la sélection d'utilisateur ;
**caractérisé en ce que** : la pluralité de modèles de délivrance de bolus pré-programmés programmés sont adaptés à des repas contenant différents aliments possédant plus d'un indice glycémique ; et au moins l'un de la pluralité de modèles de délivrance de bolus pré-programmés est recommandé à l'utilisateur pour sélection sur la base de paramètres entrés associés à l'utilisateur qui sont en fonction de caractéristiques nutritionnelles d'un repas envisagé comprenant différents aliments possédant plus d'un indice glycémique, avec l'indice glycémique des différents aliments du repas envisagé étant entrés l'un après l'autre.
